**Europäisches Patentamt**

**European Patent Office**

**Office européen des brevets**

(19)

(11) Publication number : **0 400 770 B1**

(12) # EUROPEAN PATENT SPECIFICATION

(45) Date of publication of patent specification :
**02.11.94 Bulletin 94/44**

(51) Int. Cl.$^5$ : **G01N 29/18**

(21) Application number : **90300436.4**

(22) Date of filing : **16.01.90**

(54) **Measuring the strength of a material within a moving web.**

(30) Priority : **01.06.89 US 359536**

(43) Date of publication of application :
**05.12.90 Bulletin 90/49**

(45) Publication of the grant of the patent :
**02.11.94 Bulletin 94/44**

(84) Designated Contracting States :
**BE DE FR GB IT SE**

(56) References cited :
**US-A- 4 622 853**
**US-A- 4 674 332**
**MESURES. REGULATION AUTOMATISME vol.
49, no. 8, May 1984, pp. 73-77, Paris, FR ; "CND
: des ultrasons sans contact ..."
IEEE, ULTRASONICS SYMPOSIUM vol. 1,
1986, pp. 515-526 ; P. CIELO et al.: "Laser
generation of convergent acoustic waves and
applications to materials evaluation"**

(73) Proprietor : **INTERNATIONAL CONTROL
AUTOMATION FINANCE S.A.
16 Rue des Bains
Ville de Luxembourg (LU)**

(72) Inventor : **Keyes, Marion Alvah, IV
120 Riverstone Drive
Chagrin Falls, Ohio 44022 (US)**
Inventor : **Thompson, William Lee
15364 Gar Highway
Montville, Ohio 44064 (US)**

(74) Representative : **Cotter, Ivan John et al
D. YOUNG & CO.
21 New Fetter Lane
London EC4A 1DA (GB)**

## Description

This invention relates to measuring the strength of material within a web.

A major quality consideration for the production of sheet materials, such as paper, is strength. Until recently, all strength measurements with respect to such sheet materials were made by off-line laboratory measurements. Recently, on-line measurements have been introduced using contacting gauging techniques that rely on the relationship between Young's Modulus and the speed of sound according to the equation:

$$Y = k_1.s^2$$

where $k_1$ is a function of the density of the material and s is the speed of sound within the material.

Methods proposed by Baum and Habeger, as set forth in US Patent No. US-A-4 291 577, and others, rely on rotating wheels which contact a moving web of paper or other material whose strength is being measured. The wheels contain piezoelectric or magnetostrictive transducers in their outer peripheries to create a localised contraction and expansion in the moving web of material. This contraction and expansion creates a sonic wave that travels radially from the spot of creation. Measuring the speed of sound within the material, which is the reciprocal of the transit time between two points of known separation, is used in conjunction with the density of the material to provide a measurement of the strength of the material. This approach has some inherent disadvantages, among which are that the required commutation and mechanical contact produce a signal that contains a significant amount of noise, the rotating wheels are prone to fail, mechanical structures are inevitably more costly and have more parts than electronic devices, the direct contact of the wheels with the material limits the measurement of strength to a single direction (either across the web or along the web), and mechanical methods with slippage and commutation are inherently less accurate than non-mechanical methods.

Photoacoustic interaction has been used to induce ultrasonic waves into a continuous, fast moving web of paper. US Patent No. US-A-4 674 332 (Pace et al) discloses the use of a nitrogen laser to illuminate paper with high power ultraviolet pulses. A portion of this optical energy is converted into heat, creating an acoustic wave from the resulting thermal expansion. A contacting, ultrasonic sensor or a non-contacting microphone positioned on the opposite side of the paper receives the acoustic wave and provides an indication of the speed of sound through the paper, which can be utilised to determine the strength of the paper in its direction of movement.

Another application of a laser to generate acoustic waves in paper is disclosed in US Patent No. US-A-4 622 853 (Leugers). The apparatus disclosed in this document utilises a Neodymium/YttriumAluminium-Garnet (Nd/YAG) laser with a frequency doubler to illuminate a spot on a moving web of paper. An ultrasonic wave in the paper is detected by an ultrasonic transducer in contact with the paper or by a microphone not in contact with the paper.

Because of the disadvantages that are inherent in a measuring system that requires contact with the material whose strength is being measured, it has become desirable to develop an on-line measuring system that does not require such contact.

A paper by P Cielo et al entitled "Laser Generation of Convergent Acoustic Waves and Applications to Materials Evaluation", which appears at pages 515 to 526 of Vol. 1 of the Proceedings of the IEEE 1986 Ultrasonics Symposium, discloses a technique for non-destructive testing and characterisation of industrial materials involving production of focused acoustic waves by irradiating the material with a pulsed laser beam through a suitable combination of spherical and conical lenses. The resultant ultrasonic pulse in the material reaches a relatively high amplitude in the centre of convergence, where it is probed by a non-contact interferometric optical sensor comprising a second laser and an associated detector. Mention is made of the possibility of measuring acoustic velocity in the material. The technique can be applied to ultrasonic characterisation of ceramic materials, coating thickness evaluation, detection of delaminations in layered materials, and crack inspection.

Mesures. Regulation Automatisme, Vol. 49, No. 8, 14 May 1984, pages 73 to 77, "CND: des ultrasons sans contact" discloses another technique for detection of cracks (flaws) in workpieces in which acoustic waves are generated by a laser and detected by a non-contact interferometric optical sensor comprising a second laser and an associated detector.

According to a first aspect of the invention there is provided a system for measuring the strength of material within a moving web without contacting the web, the system comprising a first light beam source having a pulsed output and arranged such that each light beam pulse therefrom contacts the material and induces an ultrasonic wave therein, a second light beam source arranged such that a light beam therefrom illuminates the material at a position spaced by a predetermined distance from a point of inducement of said ultrasonic wave within said material, light sensing means operative to detect light from the second light beam source reflected from the material so as thereby to produce a detection pulse as said ultrasonic wave traverses said position at which the light beam from the second light beam source illuminates the material, means for determining the velocity of said ultrasonic wave within the material from the lapse of time between pulsing of the first light beam source and production of said detection pulse by the light sensing means, means for determining the density of the material, means for determining the strength of the material based on the determined velocity of said ultrasonic wave within the material and the determined density of the material, and means responsive to the magnitude of said detection pulses to vary the magnitude of the light beam pulses from the first light beam source.

According to a second aspect of the invention there is provided a system for measuring the strength of material within a moving web without contacting the web, the system comprising a first light beam source having a pulsed output and arranged such that each light beam pulse therefrom contacts the material and induces an ultrasonic wave therein, two second light beam sources arranged such that light beams therefrom illuminate the material at respective positions spaced apart in the path of travel of the ultrasonic wave, a respective light sensing means associated with each of the second light beam sources and operative to detect light from the associated second light beam source reflected from the material so as thereby to produce a detection pulse as said ultrasonic wave traverses said position at which the light beam from the associated second light beam source illuminates the material, means for determining the velocity of said ultrasonic wave within the material from the lapse of time between production of said detection pulses by the respective light sensing means, means for determining the density of the material, means for determining the strength of the material based on the determined velocity of said ultrasonic wave within the material and the determined density of the material, and means responsive to the magnitude of said detection pulses to vary the magnitude of the light beam pulses from the first light beam source.

According to a third aspect of the invention there is provided a method of measuring the strength of material within a moving web without contacting the web, the method comprising the steps of:

directing a pulsed first light beam towards the material to cause an ultrasonic wave to be induced therein by each pulse;

illuminating the material with a second light beam at a position spaced by a predetermined distance from a point of inducement of the ultrasonic wave within the material;

sensing light from the second light beam reflected from the material so as thereby to produce a detection pulse as the ultrasonic wave traverses said position at which the second light beam illuminates the material;

determining the velocity of said ultrasonic wave within the material from the lapse of time between a pulse of the first light beam and production of said detection pulse;

determining the density of the material;

determining the strength of the material based on the determined velocity of the ultrasonic wave within the material and the determined density of the material; and

varying the magnitude of the pulses of the first light beam in response to the magnitude of the detection pulses.

According to a fourth aspect of the invention there is provided a method of measuring the strength of material within a moving web without contacting the web, the method comprising the steps of:

directing a pulsed first light beam towards the material to cause an ultrasonic wave to be induced therein by each pulse;

illuminating the material with two second light beams at respective positions spaced apart in the path of travel of the ultrasonic wave;

sensing light from the second light beams reflected from the material so as thereby to produce detection pulses as the ultrasonic wave traverses said positions at which the second light beams illuminate the material;

determining the velocity of said ultrasonic wave within the material from the lapse of time between production of said detection pulses by the ultrasonic wave traversing said positions;

determining the density of the material;

determining the strength of the material based on the determined velocity of the ultrasonic wave within the material and the determined density of the material; and

varying the magnitude of the pulses of the first light beam in response to the magnitude of the detection pulses.

Preferred embodiments of the invention described hereinbelow solve or at least alleviate the above-mentioned problems associated with the prior art by providing an on-line system that measures the strength of material within a moving web without contacting same. The foregoing is accomplished by utilising a first (pulsed) laser source and one or alternatively two second laser sources having beams which intercept the web of material whose strength is being measured. The first laser source generates localised spot heating which creates thermal expansion in the web of material. This thermal expansion creates an ultrasonic wave which propagates through the web in a radial direction. The wave causes the beam from the or each second laser source to be reflected by the material and the or each reflected beam is intercepted by a light sensor. By calculating the elapsed time between the transmission of the first beam into the material and the receipt of the second beam by the light sensor, or the elapsed time between receipt of the second beams by the respective light sensors, the velocity of the ultrasonic wave within the material can be determined. The velocity of the ultrasonic wave is then combined with a measurement of the density of the material to determine the strength of the material.

Further, the magnitude of the pulses produced by the first (pulsed) laser source is varied in accordance with the magnitude of detection pulses produced by the reflection of the light from the second laser source, whereby it can be ensured that the pulses from the first laser source are of a sufficient magnitude for detection purposes.

The invention will now be further described, by way of illustrative and non-limiting example, with reference to the accompanying drawings, in which similar references designate similar items throughout, and in which:

Figure 1 is a schematic diagram of a strength measuring system embodying the invention;

Figure 2 is a schematic diagram of a control circuit utilised by the measuring system shown in Figure 1;

Figure 3 is a schematic diagram of an alternative strength measuring system embodying the invention which utilises two light sensors; and

Figure 4 is a schematic diagram of a control circuit utilised by the measuring system shown in Figure 3.

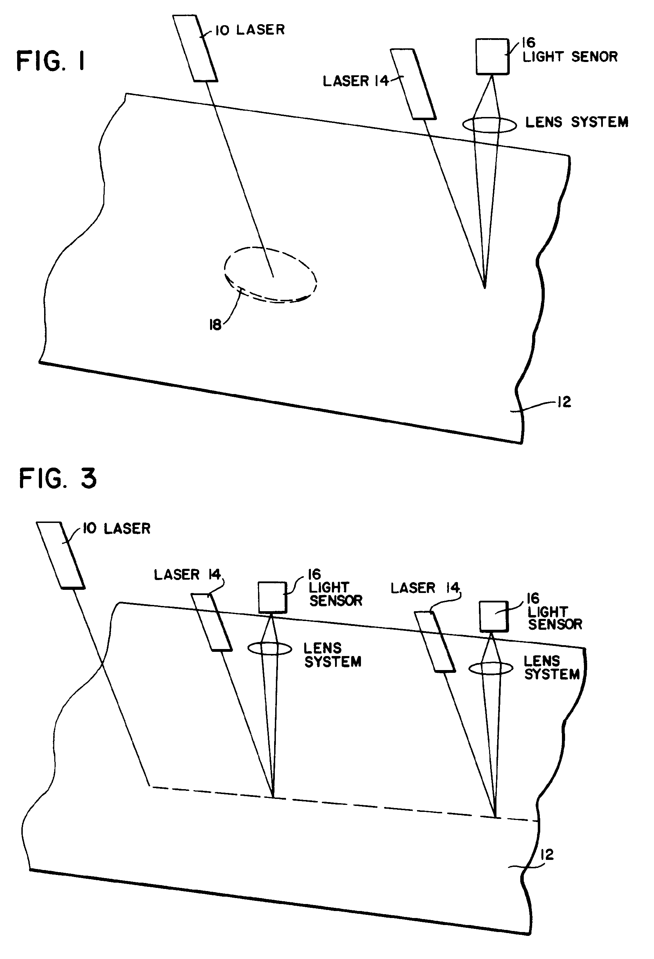

Figure 1 is a schematic diagram of a strength measuring system embodying the invention that includes a first laser source 10 directed towards a web 12 of material whose strength is to be measured, a second laser source 14 similarly directed towards the web 12, and a light sensor 16 located adjacent the second laser source 14 and positioned so that its focal point is coincident with the point of impingement of the beam from the second laser source 14 on the web 12. The distance "d" between the points of impingement of the beams from the laser sources 10 and 14 on the web 12 is known.

The present system utilises an ultrasonic wave pattern induced into the moving web 12 of material by pulses produced by the first laser source 10, which generates localised spot heating. Such localised spot heating creates thermal expansion in the material whose strength is being measured. This expansion perturbation creates a wave which propagates through the web 12 of material in a radial direction, giving an elliptical wavefront 18, due to anisotropy of the wave velocity with the direction of web movement. Measurement of wave velocity in a particular direction can be utilised to determine the strength of the material in that direction.

The light sensor 16 measures the wavefront 18 by viewing the crests and valleys of the ultrasonic wave as it passes thereunder. Separate viewing laser and light detection systems may be used for the cross-travel direction and the width-travel direction, or a single system may be scanned to read both directions. The time of arrival of the sensed pulse is compared with the time of impingement of the beam from the first laser source 10 into the web 12 of material, and the difference in time is utilised to calculate the speed of sound within the material. The foregoing speed of sound is then used in conjunction with the density of the material to determine the strength of the material being tested. The foregoing system operates at the speed of light and, as such, any time delays are inconsequential.

The first laser source 10 is a $CO_2$ laser having an output power of approximately 5.5 watts and is typically pulsed at a rate of 10 pulses per second, producing a pulse having a width of approximately 100 microseconds or less. The second laser source 14 is a HeNe laser which has an output power of approximately 2 milliwatts and which is operated continuously. The light sensor 16 can be a silicon photo-detector producing an output in the millivolt range.

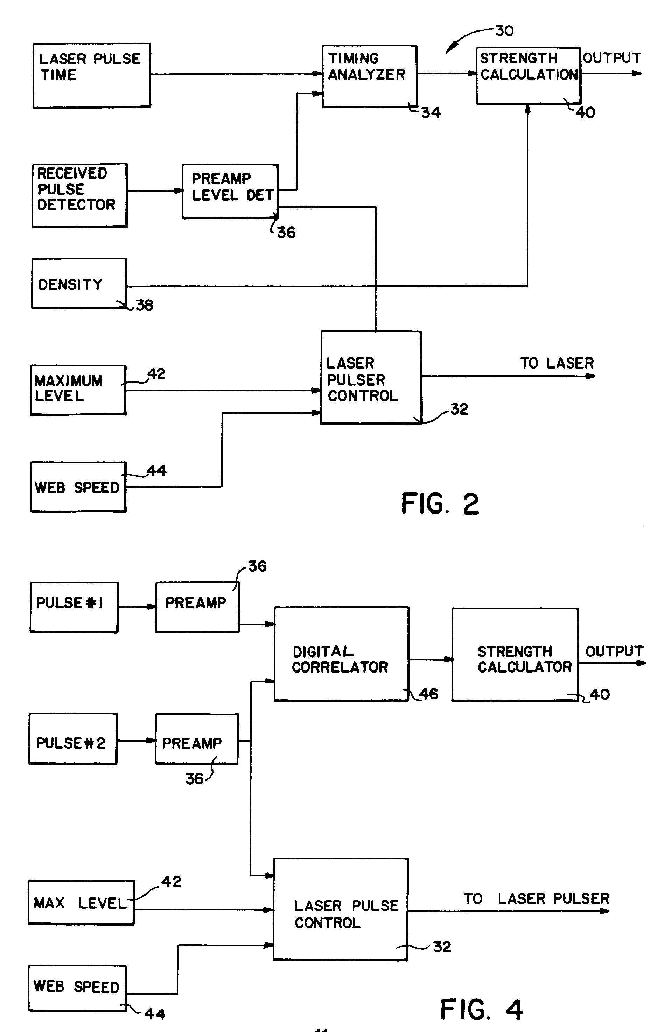

Figure 2 is a schematic diagram of a control circuit 30 of the measuring system. The control circuit 30 includes a laser pulser control 32 which regulates the operation of the first laser source 10. Each time the first laser source 10 is pulsed, a first timing pulse is transmitted by the laser pulser control 32 to a timing analyser 34. After the resulting ultrasonic wave caused by the pulse produced by the first laser source 10 has propagated through the material whose strength is being measured, the light sensor 16 intercepts the light emanating from the second laser source 14 and reflected by the material, and transmits a pulse to a preamplifier (preamp) level detector 36 which, in turn, transmits a second timing pulse to the timing analyser 34. An output of the preamp level detector 36 is connected to an input to the laser pulser control 32 and causes the pulser control 32 to increase or decrease the magnitude of the pulses produced by first laser source 10 so that the ultrasonic pulses detected by the light sensor 16 will be of sufficient magnitude for detection purposes. The timing analyser 34 determines the elapsed time $\Delta t$ between the transmission of the first timing pulse by the laser pulser control 32 and the receipt of the second timing pulse from the preamp level detector 36. The elapsed time $\Delta t$ and the known distance d between the points of impingement on the web 12 of the beams emanating from the laser sources 10 and 14 are then combined in a strength calculation device 40 with a measurement of material density provided by a density measuring device 38 to determine the velocity v of the ultrasonic wave within the material whose strength is being measured. An appropriate density measuring device 38 is disclosed in US Patent No. US-A-3 586 601 entitled "Basis Weight Control System for a Paper Making Machine. "The strength calculating device 40, which can be a microprocessor, determines the strength of the material since material strength is proportional to $k_1 v^2$, where v is the speed of sound in the material.

A maximum laser level control 42 and a web speed control 44 are provided as inputs to the laser pulser control 32. The maximum laser level control 42 ensures that the first laser source 10 causes no damage to the material whose strength is being measured and a web speed signal from the web speed control 44 allows the laser level to increase as the web speed increases.

An alternative embodiment of the invention is shown in Figure 3. In Figure 3, two sensing arrangements are employed. Inasmuch as some of the elements of Figure 3 are the same as those shown in Figure 1 and are designated by the same reference numerals, further discussion of same will not be undertaken. The main advantage of this embodiment is that the use of two sensing arrangements permits correlation of the received wave shapes using digital correlation or digital signal processing techniques so as to improve the accuracy of the resulting strength measurement and to allow for less precision and repeatability in the shape of the generated ultrasonic wave.

Figure 4 illustrates a control circuit utilised by the embodiment illustrated in Figure 3. Here again, those elements which are similar to elements shown in Figure 2 are designated by the same reference numerals and will not be discussed. The primary difference between the schematic diagram shown in Figure 4 and that shown in Figure 2 is the use of a digital correlator 46 which, as previously indicated, correlates the wave shapes received by the light sensors 16 and utilises digital correlation or digital signal processing techniques to determine the time required for the ultrasonic wave shape to traverse the web of material. Since two light sensors are utilised in the embodiment shown in Figures 3 and 4, it is possible to use less precise and less expensive pulse sources to induce the waves in the moving web 12 of material.

Each of the above-described embodiments of the invention provides the following advantages:

1) the measuring system is on-line and does not contact the web of material;

2) the system has omnidirectional measurement capability;

3) material strength can be determined across the entire web of material;

4) the system is adaptable to rough or hot material surfaces;

5) the system can utilise digital signal processing techniques; and

6) power levels are variable in order to optimise operation of the system without causing damage to the web of material.

## Claims

1. A system for measuring the strength of material within a moving web (12) without contacting the web, the system comprising a first light beam source (10) having a pulsed output and arranged such that each light beam pulse therefrom contacts the material and induces an ultrasonic wave therein, a second light beam source (14) arranged such that a light beam therefrom illuminates the material at a position spaced by a predetermined distance from a point of inducement of said ultrasonic wave within said material, light sensing means (16) operative to detect light from the second light beam source (14) reflected from the material so as thereby to produce a detection pulse as said ultrasonic wave traverses said position at which the light beam from the second light beam source (14) illuminates the material, means (34) for determining the velocity of said ultrasonic wave within the material from the lapse of time between pulsing of the first light beam source (10) and production of said detection pulse by the light sensing means (16), means (38) for determining the density of the material, means (40) for determining the strength of the material based on the determined velocity of said ultrasonic wave within the material and the determined density of the material, and means (36, 32) responsive to the magnitude of said detection pulses to vary the magnitude of the light beam pulses from the first light beam source (10).

2. A system according to claim 1, wherein: pulser control means (32) is provided for pulsing said first light beam source (10) and establishing a signal indicative thereof; said means (34) for determining the velocity of said ultrasonic wave within the material comprises timing means in communication with the pulser control means (32) and the light sensing means (16) for determining elapsed time between pulsing of the first light beam source and production of said detection pulse; preamp level detecting means (36) is responsive to said detection pulse to transmit a timing pulse to the timing means (34) to indicate ending of said elapsed time; and said preamp level detecting means (36) is connected to the pulser control means (32) to carry out said variation of the magnitude of the light beam pulses from the first light beam source (10).

3. A system according to claim 1 or claim 2, wherein the second light beam source (14) comprises a laser having a continuous output.

4. A system for measuring the strength of material within a moving web (12) without contacting the web, the system comprising a first light beam source (10) having a pulsed output and arranged such that each light beam pulse therefrom contacts the material and induces an ultrasonic wave therein, two second light beam sources (14) arranged such that light beams therefrom illuminate the material at respective positions spaced apart in the path of travel of the ultrasonic wave, a respective light sensing means (16) associated with each of the second light beam sources (14) and operative to detect light from the associated second light beam source (14) reflected from the material so as thereby to produce a detection pulse as said ultrasonic wave traverses said position at which the light beam from the associated second light beam source (14) illuminates the material, means (46) for determining the velocity of said ultrasonic wave within the material from the lapse of time between production of said detection pulses by the respective light sensing means (16), means (38) for determining the density of the material, means (40) for determining the strength of the material based on the determined velocity of said ultrasonic wave within the material and the determined density of the material, and means (36, 32) responsive to the magnitude of said detection pulses to vary the magnitude of the light beam pulses from the first light beam source (10).

5. A system according to claim 4, whereon the means (46) for determining the velocity of said ultrasonic wave within the material comprises a digital correlator for correlating the outputs of the two light sensing means (16).

6. A system according to claim 4 or claim 5, wherein each second light beam source (14) comprises a laser having a continuous output.

7. A system according to any one of the preceding claims, wherein the first light beam source (10) is a laser.

8. A method of measuring the strength of material within a moving web (12) without contacting the web, the method comprising the steps of:
   directing (10) a pulsed first light beam towards the material to cause an ultrasonic wave to be induced therein by each pulse;
   illuminating the material with a second light beam at a position spaced by a predetermined distance from a point of inducement of the ultrasonic wave within the material;
   sensing light from the second light beam reflected from the material so as thereby to produce a detection pulse as the ultrasonic wave traverses said position at which the second light beam illuminates the material;
   determining the velocity of said ultrasonic wave within the material from the lapse of time between a pulse of the first light beam and production of said detection pulse;
   determining the density of the material;

determining the strength of the material based on the determined velocity of the ultrasonic wave within the material and the determined density of the material; and
   varying the magnitude of the pulses of the first light beam in response to the magnitude of the detection pulses.

9. A method of measuring the strength of material within a moving web (12) without contacting the web, the method comprising the steps of:
   directing (10) a pulsed first light beam towards the material to cause an ultrasonic wave to be induced therein by each pulse;
   illuminating the material with two second light beams at respective positions spaced apart in the path of travel of the ultrasonic wave;
   sensing light from the second light beams reflected from the material so as thereby to produce detection pulses as the ultrasonic wave traverses said positions at which the second light beams illuminate the material;
   determining the velocity of said ultrasonic wave within the material from the lapse of time between production of said detection pulses by the ultrasonic wave traversing said positions;
   determining the density of the material;
   determining the strength of the material based on the determined velocity of the ultrasonic wave within the material and the determined density of the material; and
   varying the magnitude of the pulses of the first light beam in response to the magnitude of the detection pulses.

**Patentansprüche**

1. System zur Messung der Materialfestigkeit in einer sich bewegenden Bahn (12) ohne Berührung der Bahn mit einer ersten Lichtstrahlquelle (10), die eine pulsierende Abgabe hat und derart angeordnet ist, daß jeder Lichtstrahlimpuls von ihr in Berührung mit dem Material tritt und darin eine Ultraschallwelle erzeugt, einer zweiten Lichtstrahlquelle (14), die derart angeordnet ist, daß ein Lichtstrahl von ihr das Material in einer Position beleuchtet, die in einem vorbestimmten Abstand von einem Punkt der Erzeugung der Ultraschallwelle in dem Material entfernt ist, einer Lichtabfühleinrichtung (16), die so arbeitet daß sie Licht von der zweiten Lichtstrahlquelle (14) feststellt, das so von dem Material reflektiert wird, daß dabei ein Feststellungsimpuls erzeugt wird, wenn die Ultraschallquelle diese Position durchquert, in welcher der Lichtstrahl von der zweiten Lichtstrahlquelle (14) das Material beleuchtet, Einrichtungen (34) zur Bestimmung der Geschwindigkeit der Ultraschallwelle in dem Material aufgrund der Zeit, die zwischen dem Pulsieren der ersten Lichtstrahlquelle (10) und der Erzeugung des Feststellungsimpulses durch die Lichtabfühleinrichtung (16) vergeht, Einrichtungen (38) zur Bestimmung der Dichte des Materials, Einrichtungen (40) zur Bestimmung der Festigkeit des Materials auf der Grundlage der ermittelten Geschwindigkeit der Ultraschallwelle in dem Material und der ermittelten Dichte des Materials sowie Einrichtungen (36, 32), die auf die Größe der Feststellungsimpulse ansprechen, um die Größe der Lichtstrahlimpulse von der ersten Lichtstrahlquelle (10) zu variieren.

2. System nach Anspruch 1, bei dem eine Impulsgebersteuerung (32) für das Pulsieren der ersten Lichtstrahlquelle (10) und zur Erzeugung eines sie angebenden Signales vorgesehen ist, die Einrichtung (34) zur Bestimmung der Geschwindigkeit der Ultraschallwelle in dem Material eine Zeitmeßeinrichtung in Verbindung mit der Impulsgebersteuereinrichtung (32) und der Lichtabfühleinrichtung (16) zur Bestimmung der Zeit, die zwischen dem Pulsieren der ersten Lichtstrahlquelle und der Erzeugung des Feststellungsimpulses vergeht, umfaßt, ein Vorverstärkerdetektor (36) auf den Feststellungsimpuls anspricht, um einen Zeitmeßimpuls zu der Zeitmeßeinrichtung (34) zu übertragen, um das Ende der verstrichenen Zeit anzugeben, und der Vorverstärkerdetektor (36) mit der Impulsgebersteuereinrichtung (32) verbunden ist, um das Variieren der Größe der Lichtstrahlimpulse von der ersten Lichtstrahlquelle (10) auszuführen.

3. System nach Anspruch 1 oder Anspruch 2, bei dem die zweite Lichtstrahlquelle (14) einen Laser mit einer kontinuierlichen Abgabe umfaßt.

4. System zur Messung der Materialfestigkeit in einer sich bewegenden Bahn (12) ohne Berührung der Bahn mit einer ersten Lichtstrahlquelle (10), die eine pulsierende Abgabe hat und derart angeordnet ist, daß jeder Lichtstrahlimpuls von ihr das Material berührt und eine Ultraschallwelle darin erzeugt, zwei zweiten Lichtstrahlquellen (14), die derart angeordnet sind, daß die Lichtstrahlen von ihnen das Material in entsprechenden voneinander beabstandeten Positionen auf dem Wanderweg der Ultraschallwelle beleuchten, einer jeweiligen Lichtabfühleinrichtung (16), die mit jeder der zweiten Lichtstrahlquellen (14) verbunden ist und derart arbeitet, daß sie Licht von der verbundenen zweiten Lichtstrahlquelle (14) feststellt, das von dem Material derart reflektiert wird, daß dabei ein Feststellungsimpuls erzeugt wird, wenn die Ultraschallwelle diese Position durchquert, in welcher der Lichtstrahl von der verbundenen zweiten Lichtstrahlquelle (14) das Material beleuchtet, Einrichtungen (46) zur Bestimmung der Geschwindigkeit der Ultraschallwelle in dem Material aufgrund der Zeit, die zwischen der Erzeugung der Feststellungsimpulse durch die betreffende Lichtabfühleinrichtung (16) verstreicht, Einrichtungen (38) zur Bestimmung der Dichte des Materials, Einrichtungen (40) zur Bestimmung der Festigkeit des Materials auf der Grundlage der ermittelten Geschwindigkeit der Ultraschallwelle in dem Material und der ermittelten Dichte des Materials sowie Einrichtungen (36, 32), die auf die Größe der Feststellungsimpulse ansprechen, um die Größe der Lichtstrahlimpulse von der ersten Lichtstrahlquelle (10) zu variieren.

5. System nach Anspruch 4, bei dem die Einrichtungen (46) zur Bestimmung der Geschwindigkeit der Ultraschallwelle in dem Material einen digitalen Korrelator umfaßt, um die Ausgänge der beiden Lichtabfühleinrichtungen (16) in Wechselbeziehung zueinander zu bringen.

6. System nach Anspruch 4 oder Anspruch 5, bei dem jede zweite Lichtstrahlquelle (14) einen Laser mit einer kontinuierlichen Abgabe umfaßt.

7. System nach einem der vorausgehenden Ansprüche, bei dem die erste Lichtstrahlquelle (10) ein Laser ist.

8. Verfahren zur Messung der Materialfestigkeit in einer sich bewegenden Bahn (12) ohne Berührung der Bahn mit den Stufen, in denen man einen pulsierenden ersten Lichtstrahl auf das Material richtet, um eine darin durch jeden Impuls zu erzeugende Ultraschallwelle zu verursachen, das Material mit einem zweiten Lichtstrahl in einer Position beleuchtet, die in einem vorbestimmten Abstand von dem Punkt der Erzeugung der Ultraschallwelle in dem Material entfernt ist, Licht von dem zweiten Lichtstrahl abfühlt, der von dem Material derart reflektiert wird, daß dabei ein Feststellungsimpuls erzeugt wird, wenn die Ultraschallwelle diese Position durchquert, in welcher der zweite Lichtstrahl das Material beleuchtet, die Geschwindigkeit der Ultraschallwelle in dem Material aufgrund der Zeit bestimmt, die zwischen einem Impuls des ersten Lichtstrahles und der Erzeugung des Feststellungsimpulses vergeht, die Dichte des Materials bestimmt, die Festigkeit des Materials auf der Grundlage der ermittelten Geschwindigkeit der Ultraschallwelle in dem Material und der ermittelten Dichte des Materials bestimmt und die Größe der Impulse des ersten Lichtstrahles in Reaktion auf die Größe der Feststellungsimpulse variiert.

9. Verfahren zur Messung der Materialfestigkeit in einer sich bewegenden Bahn (12) ohne Berührung der Bahn mit den Stufen, in denen man einen pulsierenden ersten Lichtstrahl auf das Material richtet, um eine darin durch jeden Impuls zu erzeugende Ultraschallwelle zu verursachen, das Material mit zwei zweiten Lichtstrahlen in entsprechenden Positionen, die auf dem Wanderweg der Ultraschallwelle voneinander beabstandet sind, beleuchtet,
Licht von den zweiten Lichtstrahlen abfühlt, die von dem Material derart reflektiert werden, daß dabei Feststellungsimpulse erzeugt werden, wenn die Ultraschallwelle diese Positionen durchquert, in welchen die zweiten Lichtstrahlen das Material beleuchten,
die Geschwindigkeit der Ultraschallwelle in dem Material aufgrund der Zeit bestimmt, die zwischen der Erzeugung der Feststellungsimpulse durch die diese Positionen durchquerende Ultraschallwelle verstreicht,
die Dichte des Materials bestimmt,
die Festigkeit des Materials aufgrund der ermittelten Geschwindigkeit der Ultraschallwelle in dem Material und der ermittelten Dichte des Materials bestimmt und die Größe der Impulse des ersten Lichtstrahles in Reaktion auf die Größe der Feststellungsimpulse variiert.

**Revendications**

1. Système pour mesurer la résistance mécanique d'une matière à l'intérieur d'une bande en mouvement (12) sans contacter la bande, le système comprenant une première source de rayon lumineux (10) ayant une sortie pulsée et agencée de telle manière que chaque impulsion de rayon lumineux qui en est issue contacte la matière et y induit une onde ultrasonore ; une seconde source de rayon lumineux (14) agencée de telle façon qu'un rayon lumineux qui en est issu éclaire la matière au droit d'une position écartée d'une distance prédéterminée du point d'induction de ladite onde ultrasonore dans ladite matière ; un moyen de détection de lumière (16) servant à détecter la lumière provenant de la seconde source de rayon lumineux (14) réfléchie par la matière, de façon à produire ainsi une impulsion de détection lorsque ladite onde ultrasonore passe devant ladite position à laquelle le rayon lumineux issu de la seconde source de rayon lumineux (14) éclaire la matière ; un moyen (34) pour déterminer la vitesse de ladite onde ultrasonore dans la matière à partir du temps écoulé entre le moment où la première source de rayon lumineux (10) a été impulsée et la production de ladite impulsion de détection par le moyen de détection de lumière (16) ; un moyen (38) pour déterminer la densité de la matière ; un moyen (40) pour déterminer la résistance mécanique de la matière en se basant sur la vitesse déterminée de ladite onde ultrasonore dans la matière et sur la densité déterminée de la matière ; et un moyen (36, 32) sensible à l'amplitude desdites impulsions de détection pour faire varier l'amplitude des impulsions de rayon lumineux issues de la première source de rayon lumineux (10).

2. Système selon la revendication 1, dans lequel : un moyen de commande d'impulseur (32) est prévu pour pulser ladite première source de rayon lumineux (10) et pour établir un signal indicatif de cela ; dans lequel ledit moyen (34) pour déterminer la vitesse de ladite onde ultrasonore dans la matière comprend un moyen de cadencement en communication avec le moyen de commande d'impulseur (32) et le moyen de détection de lumière (16), pour déterminer le temps écoulé entre l'émission de l'impulsion de la première source de rayon lumineux et la production de ladite impulsion de détection ; un moyen de détection de niveau de préamplification (36) en réponse à ladite impulsion de détection pour émettre une impulsion de cadencement vers le moyen de cadencement (34) pour indiquer la fin dudit temps écoulé ; et dans lequel ledit moyen de détection de niveau de préamplification (36) est connecté au moyen de commande d'impulseur (32) pour effectuer ladite variation de l'amplitude des impulsions de rayon lumineux issues de la première source de rayon lumineux (10).

3. Système selon la revendication 1 ou la revendication 2, dans lequel la seconde source de rayon lumineux (14) comprend un laser ayant une sortie continue.

4. Système pour mesurer la résistance mécanique d'une matière à l'intérieur d'une bande en mouvement (12) sans contacter la bande, le système comprenant une première source de rayon lumineux (10) ayant une sortie pulsée et agencée de telle manière que chaque impulsion de rayon lumineux qui en est issue contacte la matière et y induit une onde ultrasonore ; deux sources de rayon lumineux (14) agencées de telle manière que des rayons lumineux qui en sont issus éclairent la matière au droit de positions respectives espacées dans le trajet de propagation de l'onde ultrasonore ; un moyen de détection de lumière (16) respectif associé avec chacune des secondes sources de rayon lumineux (14) et servant à détecter la lumière provenant de la seconde source de rayon lumineux (14) associée réfléchie par la matière, de façon à produire ainsi une impulsion de détection lorsque ladite onde ultrasonore passe devant ladite position à laquelle le rayon lumineux issu de la seconde source de rayon lumineux (14) associée éclaire la matière ; un moyen (46) pour déterminer la vitesse de ladite onde ultrasonore dans la matière à partir du temps écoulé entre la production desdites impulsions de détection par le moyen de détection de lumière (16) respectif ; un moyen (38) pour déterminer la densité de la matière ; un moyen (40) pour déterminer la résistance mécanique de la matière en se basant sur la vitesse déterminée de ladite onde ultrasonore dans la matière et sur la densité déterminée de la matière ; et un moyen (36, 32) sensible à l'amplitude desdites impulsions de détection pour faire varier l'amplitude des impulsions de rayon lumineux issues de la première source de rayon lumineux (10).

5. Système selon la revendication 4, dans lequel le moyen (46) pour déterminer la vitesse de ladite onde ultrasonore à l'intérieur de la matière comprend un corrélateur numérique pour corréler les sorties des deux moyens de détection de lumière (16).

6. Système selon la revendication 4 ou la revendication 5, dans lequel chaque seconde source de rayon lumineux (14) comprend un laser ayant une sortie continue.

7. Système selon l'une quelconque des revendications précédentes, dans lequel la première source de rayon lumineux (10) est un laser.

8. Procédé de mesure de la résistance mécanique de la matière à l'intérieur d'une bande en mouvement (12) sans contacter la bande, le procédé comprenant les étapes :

de projection (10) d'un premier rayon lumineux pulsé en direction de la matière pour faire en sorte qu'une onde ultrasonore y soit induite par chaque impulsion ;

d'éclairement de la matière avec un second rayon lumineux au droit d'une position espacée d'une distance prédéterminée par rapport au point d'induction de l'onde ultrasonore dans la matière ;

de détection de la lumière issue du second rayon lumineux réfléchi à partir de la matière, de façon à produire par ce moyen une impulsion de détection lorsque l'onde ultrasonore passe devant ladite position à laquelle le second rayon lumineux éclaire la matière ;

de détermination de la vitesse de ladite onde ultrasonore dans la matière à partir du temps écoulé entre une impulsion du premier rayon lumineux et la production de ladite impulsion de détection ;

de détermination de la densité de la matière ;

de détermination de la résistance mécanique de la matière en se basant sur la vitesse déterminée de l'onde ultrasonore dans la matière et sur la densité déterminée de la matière ; et,

de modification de l'amplitude des impulsions du premier rayon lumineux en réponse à l'amplitude des impulsions de détection.

9. Procédé de mesure de la résistance mécanique de la matière à l'intérieur d'une bande en mouvement (12) sans contacter la bande, le procédé comprenant les étapes :

de projection (10) d'un premier rayon lumineux pulsé en direction de la matière pour faire en sorte qu'une onde ultrasonore y soit induite par chaque impulsion ;

d'éclairement de la matière avec deux seconds rayons lumineux au droit de positions respectives espacées dans le trajet de propagation de l'onde ultrasonore ;

de détection de la lumière issue des seconds rayons lumineux réfléchis à partir de la matière, de façon à produire par ce moyen des impulsions de détection lorsque l'onde ultrasonore passe devant lesdites positions auxquelles les seconds rayons lumineux éclairent la matière ;

de détermination de la vitesse de ladite onde ultrasonore dans la matière à partir du temps écoulé entre la production desdites impulsions de détection par le passage de l'onde ultrasonore dans lesdites positions ;

de détermination de la densité de la matière ;

de détermination de la résistance mécanique de la matière en se basant sur la vitesse déterminée de l'onde ultrasonore dans la matière et sur la densité déterminée de la matière ; et,

de modification de l'amplitude des impulsions du premier rayon lumineux en réponse à l'amplitude des impulsions de détection.

## FIG. 1

10 LASER

16 LIGHT SENOR

LASER 14

LENS SYSTEM

18

12

## FIG. 3

10 LASER

LASER 14

16 LIGHT SENSOR

LENS SYSTEM

LASER 14

16 LIGHT SENSOR

LENS SYSTEM

12

FIG. 2

FIG. 4

11